# EUROPÄISCHE PATENTSCHRIFT

(11) **EP 1 945 720 B1**
(45) Veröffentlichungstag und Bekanntmachung des Hinweises auf die Patenterteilung: **28.03.2012**
(21) Anmeldenummer: 06705877.6
(22) Anmeldetag: 31.01.2006
(51) Int. Cl.: G01N 33/68, C09B 23/10

(54) **VERFAHREN ZU NACHWEIS UND QUANTIFIZIERUNG VON PROTEINEN UNTER VERWENDUNG VON FARBSTOFFEN AUF DER BASIS VON POLYMETHINEN**
A METHOD FOR DETECTINGAND QUANTIFYING PROTEINS USING POLYMETHINE-BASED DYES
PROCEDE DE DETECTIONET QUANTIFICATION DE PROTEINES UTILISANT DES COLORANTS A BASE DE POLYMETHINES

(30) Priorität: 31.01.2005 DE 102005004745
(43) Veröffentlichungstag der Anmeldung: 23.07.2008
(73) Patentinhaber: Dyomics GmbH, 07745 Jena (DE)
(72) Erfinder: WENZEL, Matthias, 07743 Jena (DE); CZERNEY, Peter, 99425 Weimar (DE); SCHWEDER, Bernd, 07743 Jena (DE); LEHMANN, Frank, 07749 Jena (DE)
(74) Vertreter: Oehmke, Volker
(86) Internationale Anmeldenummer: PCT/DE2006/000148
(87) Internationale Veröffentlichungsnummer: WO 2006/079334

(56) Entgegenhaltungen:
- EP-A- 1 318 177
- EP-A- 1 535 969
- WO-A2-2006/080986
- DE-A1- 10 159 078
- US-A1- 2004 260 093
- GRUMMT U-W ET AL: "New near-infrared-absorbing acidochromic dyes and their application in sensor techniques" SENSORS AND ACTUATORS B, ELSEVIER SEQUOIA S.A., LAUSANNE, CH, Bd. 39, Nr. 1-3, März 1997 (1997-03), Seiten 395-400, XP004087779 ISSN: 0925-4005
- TATIKOLOV, A.S. AND COSTA, S.M.B.: "Complexation of polymethine dyes with human serum albumin: a spectroscopic study" BIOPHYSICAL CHEMISTRY, Bd. 107, 2004, Seiten 33-49, XP002381135

## Beschreibung

### Stand der Technik

Die Quantifizierung von Proteinen ist ein wichtiges Werkzeug der Bioanalytik. Sowohl in Lösungen als auch in Gelen werden Proteinmengen mittels ihrer Interaktion mit Fluoreszenzfarbstoffen bestimmt. Beispiele dafür sind das auf einer Cyaninstruktur beruhende Albumin-Blue (Wolfbeis et al., US5182214), Cumarin basierte Cyanin-Farsbtoffe (Berkelman PCT/US2005/044378), Merocyanine (Haugland et al. US5616502) oder SYPRO Ruby (Haugland et al., Handbook of Fluorescent Probes and Research Products, 9th Edition, auch http://www.probes.com), welches sich von einem Metall-Liganden-Komplex ableitet. Durch das Anlagern der Verbindungen an die Oberfläche von Proteinen kommt es unter anderem zu einer Versteifung der molekularen Struktur des Chromophors, was zu einem drastischen Anstieg der Quantenausbeute führt. Auch die Ausbildung von Wasserstoff-Brückenbindungen und das Abschirmen gegenüber Löschung der Emission durch gelösten Sauerstoff spielen beim Anstieg des Fluoreszenzsignals eine Rolle.

Czerney et al. (EP 1318177 und im dazugehörigen Recherchebericht genannte Dokumente) beschreiben und beanspruchen reaktive Marker unter anderem auf der Basis von cumarin- bzw. chinolonbasierten Farbstoffen, die zum kovalenten Markieren von Biomolekülen geeignet sind. Dabei werden auch 4-hydroxysubstituierte Cumarinfarbstoffe offengelegt, die mit einer reaktiven Funktion versehen sind. Auf nichtkovalente Wechselwirkungen zwischen offengelegten Verbindungen und Proteinen sowie damit einhergehende Änderungen der spektralen Eigenschaften der Farbstoffe wird in jenem Dokument nicht eingegangen.

### Details der Erfindung

Die vorliegende Erfindung beschreibt ein Verfahren zu Nachweis und Quantifizierung von Proteinen unter Verwendung von Farbstoffen auf Basis von 4-substituierten, cumarin- bzw. chinolonbasierten Farbstoffen, die ihr Fluoreszenzverhalten in Gegenwart von Proteinen verändern.

Im Detail kann gezeigt werden, dass Farbstoffe auf der Basis von Polymethinen der allgemeinen Formeln **I - III** 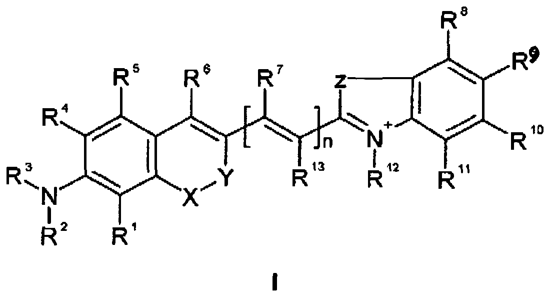 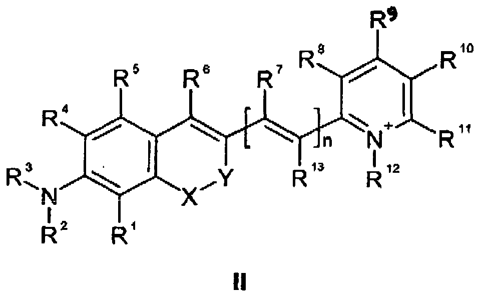 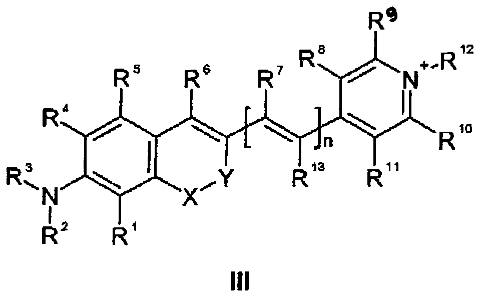 wobei R¹ sowie R⁴ - R¹³ gleich oder unterschiedlich sind und Wasserstoff, ein oder mehrere Chlor- und/oder Bromatome, Alkyl-, Aryl-, Heteroaryl-, Cycloalkyl-, Heterocycloalkyl-, Alkyloxy-, Alkylmercapto-, Aryloxy-, Arylmercapto-, Heteroaryloxy-, Heteroarylmercapto- oder Cyano-Reste, eine oder mehrere Hydroxy-, eine oder mehrere alkylsubstituierte oder cyclische Aminofunktionen und R² und R³ gleich oder unterschiedliche Alkyl-Reste mit mindestens 3 Kohlenstoffatomen sind sowie R¹ und R² und/oder R³ und R⁴ oder R² und R³ und/oder R⁸ und R⁹ und/oder R¹⁰ und R¹¹ weitere aliphatische oder aromatische Ringe bzw. zwei benachbarte Reste, z.B. in **I** bzw. **II** R¹² und R¹³ zusammen einen oder mehrere aliphatische oder aromatische Ringe bilden können,
- X-Y zusammen für ein Element aus der Gruppe O, S, Se, Te, oder das Strukturelement (CR₂)ₘ, NR oder SO₂, wobei R gleich oder unterschiedlich die Funktionen von R¹ - R¹³ einnehmen kann und m für 1 - 4, stehen können,
- X-Y für die Strukturelemente -CR₂-O-, -O-CR₂-, -CO-O-, -CO-NR- oder -NR-COwobei R gleich oder unterschiedlich die Funktionen von R¹ - R¹³ einnehmen, stehen können,
- Z für O, S, NR¹⁴ oder CR¹⁵R¹⁶ wobei R¹⁴ , R¹⁵ und R¹⁶ gleich oder unterschiedlich die Funktionen von R¹ - R¹³ stehen kann,
- n den Wert von 0 - 4 einnehmen kann,
- einer oder mehrere der Substituenten R¹ - R¹³ solubilisierende bzw. ionisierbare bzw. ionisierte Substituenten wie SO₃⁻, PO₃²⁻, CO₂⁻, O⁻, NR₃⁺, Cyclodextrin oder Zucker darstellen können, die die hydrophilen Eigenschaften der Farbstoffe bestimmen, wobei diese Substituenten auch über eine aliphatische oder heteroaliphatische, möglicherweise zyklische Spacergruppe am eigentlichen Chromophor angebunden sein können und
- die aliphatische oder heteroaliphatische Spacergruppe aus einem Strukturelement -[(CH₂)ₐ-Y-(CH₂)_{b}]_{c}-, worin Y gleich oder verschieden eine CR₂-, O-, S-, SO₂, SO₂NH-, NR-, CO₂- oder CONR- Funktion sein kann, wobei R die Funktionen von R¹ - R¹³ einnehmen kann und a und b gleich oder verschieden die Werte von 0 - 18 und c die Werte von 1- 18 darstellen,
für den fluoreszenzbasierten Nachweis und die Quantifizierung von Proteinen geeignet sind.

Insbesondere die Quantifizierung von Proteinen in Lösung (wässriger Puffer) und die Visualisierung von Proteinen in Gelen ist mit der vorliegenden Erfindung beabsichtigt.

Ein erheblicher Vorteil gegenüber herkömmlicher, auf der Verwendung von Metall-Ligand-Komlexen beruhender Visualisierung von Proteinen ist die Vermeidung des Einsatzes von schwermetallhaltigen Reagenzien.

Gegenüber dem Albumin-Blau hat die Verwendung der in dieser Erfindung beschriebenen Anfärbereagenzien den Vorteil der Kompatibilität mit in der Bioanalytik und medizinischen Diagnostik weit verbreitetem Argon-, Helium-Neon- und Dioden-Lasern.

Die Beispiele Nr. 5 bis 7 stehen für einige im Rahmen der Erfindung synthetisierte Farbstoffe zum fluoreszenzbasierten Nachweis und der Quantifizierung von Proteinen und sind in der nachfolgenden Tabelle 1 aufgeführt. Die Beispiele Nr. 1 bis 4 und 8 gehören nicht zur Erfindung. Die Absorptions- und Emissionsmaxima wurden in Methanol gemessen.

**Tabelle 1:**

| **Beispiel** | **Analytische und spektroskopische Daten** |
|---|---|
| 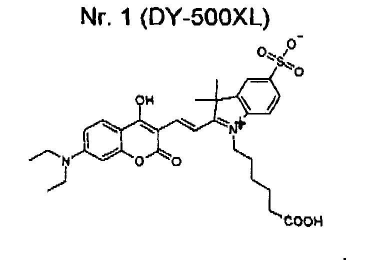 | C₃₁H₃₆N₂O₈S |
| | 596.71 g/ mol |
| | |
| | MS (ESI⁺), [m/z]: 597 [M + H]⁺; 619 |
| | [M + Na]⁺ |
| | MS (ESI ⁻), [m/z]: 595 [M - H]⁻ |
| | |
| | λₘₐₓ 505 nm |
| | λₑₘ 555 nm |
| 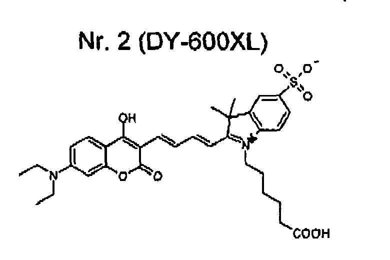 | C₃₃H₃₈N₂O₈S |
| | 622.74 g/ mol |
| | |
| | MS (ESI⁻), [m/z]: 643 [M -2H + Na]⁻; 621 (M-H]⁻ |
| | |
| | λₘₐₓ 603 nm |
| | λₑₘ 634 nm |
| 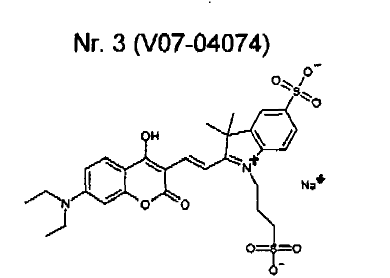 | C₂₈H₃₁N₂O₉S₂Na |
| | 622.74 g/ mol |
| | |
| | MS (ESI⁻), [m/z]: 301 [M⁻-H]²⁻; 625 |
| | [M⁻ - H + Na]⁻ |
| | |
| | λₘₐₓ 505 nm |
| | λₑₘ 572 nm |
| 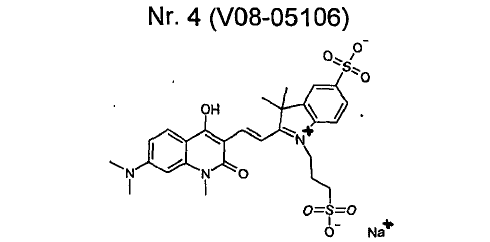 | C₂₇H₃₀N₃O₈S₂Na |
| | 611.67 g/ mol |
| | |
| | MS (ESI⁻), [m/z]: 293.6 [M]²⁻; 588 [M]⁻ |
| | |
| | λₘₐₓ 508 nm |
| | λₑₘ 560 nm |
| 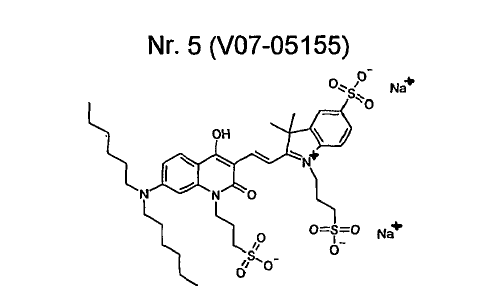 | C₃₉H₅₃N₃O₁₁S₃Na₂ |
| | 882.04 g/ mol |
| | |
| | MS (ESI⁻), [m/z]: 278.3 [M²⁻ -H]³⁻; 417 [M]²⁻ |
| | |
| | λₘₐₓ 516 nm |
| | λₑₘ 598 nm |
| 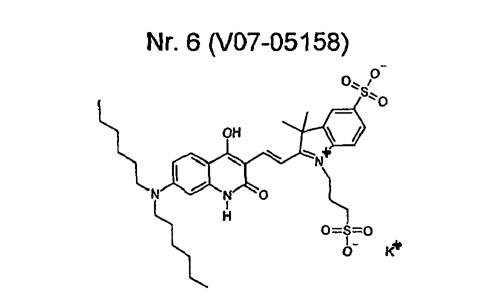 | C₃₆H₄₈N₃O₈S₂K |
| | 754.03 g/ mol |
| | |
| | MS (ESI⁻), [m/z]: 356.7 [M⁻ -H]²⁻; 714 [M]⁻ |
| | |
| | λₘₐₓ 513 nm |
| | λₑₘ 580 nm |
| 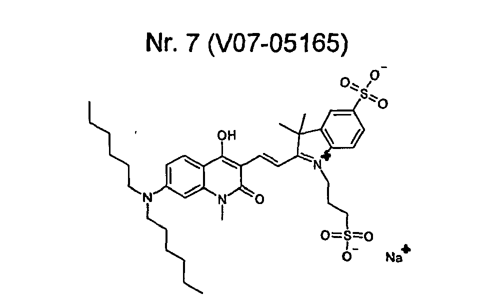 | C₃₇H₅₀N₃O₈S₂Na |
| | 751.94 g/ mol |
| | |
| | MS (ESI⁻), [m/z]: 363.8 [M⁻ -H]²⁻; 728 [M]⁻ |
| | |
| | λₘₐₓ 513 nm |
| | λₑₘ 580 nm |
| 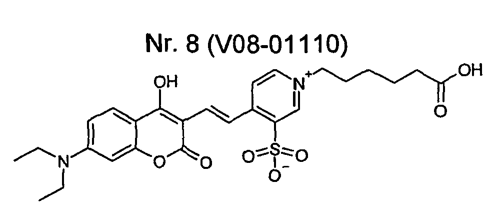 | C₂₆H₃₀N₂O₈S |
| | 530.60 g/ mol |
| | |
| | MS (ESI⁻), [m/z]: 529 [M -H]⁻ |
| | |
| | λₘₐₓ 520 nm |
| | λₑₘ 570 nm |

Die folgenden Ausführungsbeispiele demonstrieren den Gebrauch der Erfindung.

### Ausführungsbeispiel 1

Zur Bestimmung der Fluoreszenz von Protein-Farbstoff-Lösungen mit unterschiedlichen Protein-Farbstoff-Verhältnissen wurden Lösungen mit folgenden Konzentrationen eingesetzt:

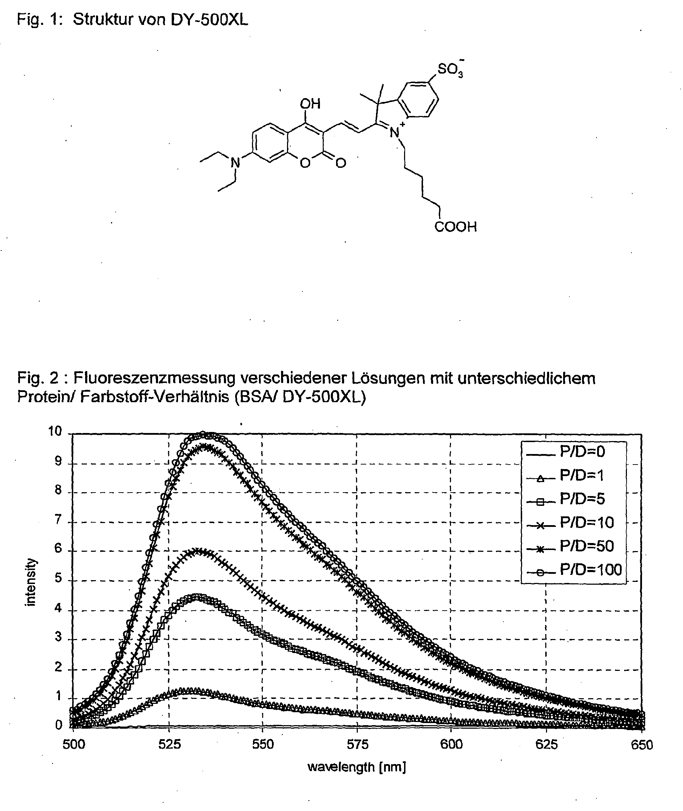

Farbstoff: Es wurde eine Lösung von DY-500XL (Fig. 1) in PBS-Puffer (22 mM, pH 7,2) der Konzentration 2*10⁻⁶ mol/l hergestellt.

Protein: Es wurden 5 verschiedene Lösungen von Rinderserumalbumin (BSA) in PBS-Puffer (siehe oben) mit folgenden Konzentrationen hergestellt:
1. 2*10⁻⁶ mol/l
2. 1*10⁻⁵ mol/l
3. 2*10⁻⁵ mol/l
4. 1*10⁻⁴ mol/l
5. 2*10⁻⁴ mol/l

Zur Messung der Fluoreszenz wurden jeweils 1,5 ml der Farbstofflösung und 1,5 ml der entsprechenden Protein-Lösung gemischt.

Begonnen wurde mit der höchsten Proteinkonzentration (Lösung 5). Damit wird ein Protein/ Farbstoff-Verhältnis von 100/1 eingestellt. Der Wert der Fluoreszenz dieser Lösung wurde als Maximum gesetzt und die nachfolgenden Werte wurden darauf bezogen.

Wie zu sehen ist (Fig. 2), zeigt der Farbstoff DY-500XL in Abwesenheit vom Protein nahezu keine Fluoreszenz. In Anwesenheit vom Protein (hier als Beispiel BSA) ist eine drastische Steigerung der Fluoreszenz zu beobachten.

### Ausführungsbeispiel 2

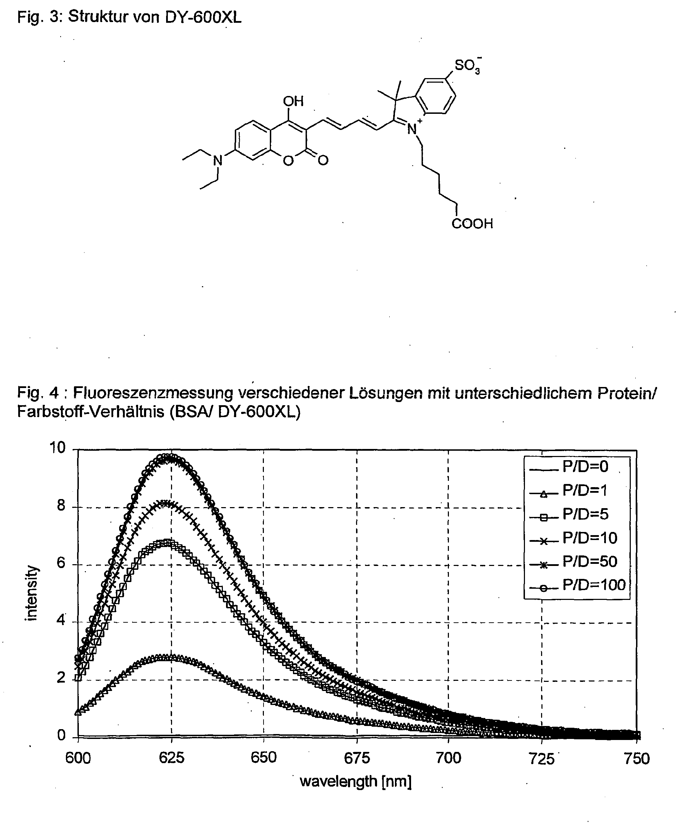

Mit dem Farbstoff DY-600XL (Struktur in Fig. 3) wurde analog zum Ausführungsbeispiel 1 verfahren. Die entsprechenden Fluoreszenzspektren sind in Fig. 4 dargestellt.

## Patentansprüche

1. Ein Verfahren zum fluoreszenzoptischen Nachweis von Proteinen **dadurch gekennzeichnet, dass** eine wässrige Mischung, die aus den nachzuweisenden Proteinen, verschiedenen Detergentien und aus einem oder mehreren Marker-Farbstoffen auf der Basis von Polymethinen der allgemeinen Formeln **I - III** 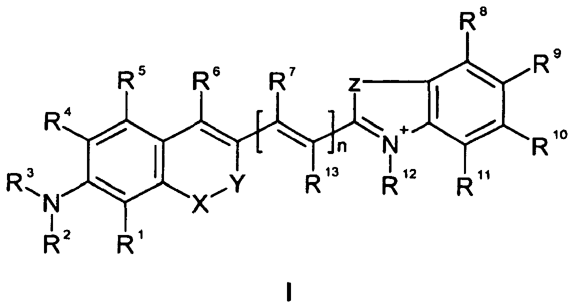 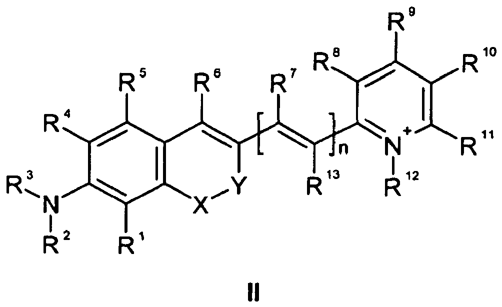 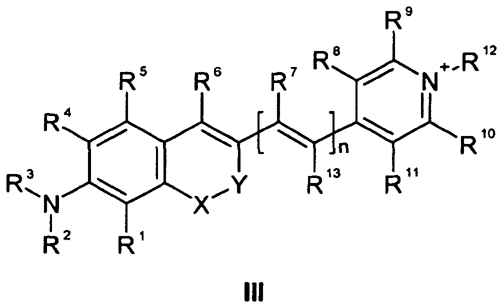
• wobei R¹ sowie R⁴ - R¹³ gleich oder unterschiedlich sind und Wasserstoff, ein oder mehrere Chlor- und/oder Bromatome, Alkyl-, Aryl-, Heteroaryl-, Cycloalkyl-, Heterocycloalkyl-, Alkyloxy-, Alkylmercapto-, Aryloxy-, Arylmercapto-, Heteroaryloxy-, Heteroarylmercapto- oder Cyano-Reste, eine oder mehrere Hydroxy-, eine oder mehrere alkylsubstituierte oder cyclische Aminofunktionen und R² und R³ gleich oder unterschiedliche Alkyl-Reste mit mindestens 3 Kohlenstoffatomen sind sowie und R¹ und R² und/oder R³ und R⁴ oder R² und R³ und/oder R⁸ und R⁹ und/oder R¹⁰ und R¹¹ weitere aliphatische oder aromatische Ringe bzw. zwei benachbarte Reste, z.B. in **I** bzw. **II** R¹² und R¹³ zusammen einen oder mehrere aliphatische oder aromatische Ringe bilden können,
• X-Y zusammen für ein Element aus der Gruppe O, S, Se, Te, oder das Strukturelement (CR₂)ₘ, NR oder SO₂, wobei R gleich oder unterschiedlich die Funktionen von R¹ - R¹³ einnehmen kann und m für 1 - 4, stehen können,
• X-Y für die Strukturelemente -CR₂-O-, -O-CR₂-, -CO-O-, -CO-NR- oder -NR-CO- wobei R gleich oder unterschiedlich die Funktionen von R¹ - R¹³ einnehmen, stehen können,
• Z in I für O, S, NR¹⁴ oder CR¹⁵R¹⁶, wobei R¹⁴ , R¹⁵ und R¹⁶ gleich oder unterschiedlich die Funktionen von R¹ - R¹³ einnehmen können, stehen kann und
• n den Wert von 0 - 4 einnehmen kann und/oder
• dass einer oder mehrere der Substituenten R¹ - R¹³ solubilisierende bzw. ionisierbare bzw. ionisierte Substituenten wie SO₃⁻, PO₃²⁻, CO₂⁻, O⁻, NR₃⁺, Cyclodextrin oder Zucker darstellen können, die die hydrophilen Eigenschaften der Farbstoffe bestimmen, wobei diese Substituenten auch über eine aliphatische oder heteroaliphatische, möglicherweise zyklische Spacergruppe am eigentlichen Chromophor angebunden sein können und
• sich dadurch auszeichnen, dass die aliphatische oder heteroaliphatische Spacergruppe aus einem Strukturelement -[(CH₂)ₐ-Y-(CH₂)_{b}]_{c}-, worin Y gleich oder verschieden eine CR₂-, O-, S-, SO₂, SO₂NH-, NR-, CO₂- oder CONR- Funktion sein kann, wobei R die Funktionen von R¹ - R¹³ einnehmen kann und a und b gleich oder verschieden die Werte von 0 - 18 und c die Werte von 1- 18 darstellen, besteht, verwendet wird.

2. Ein Verfahren nach Anspruch 1, bei der sich nach dem Zusammenbringen der Markerfarbstoffe vom Typ I - III mit dem Protein einen Marker-Protein-Komplex ausbildet, der mit Hilfe fluoreszenzoptischer Verfahren detektiert werden kann.

3. Ein Verfahren nach den Ansprüchen 1 und 2, bei der zum Nachweis von Proteinen wenigstens einer der Verbindungen Nr. 1 - 3 mit folgenden Formeln eingesetzt werden:
| Nr. | Formel |
|---|---|
| 1 | 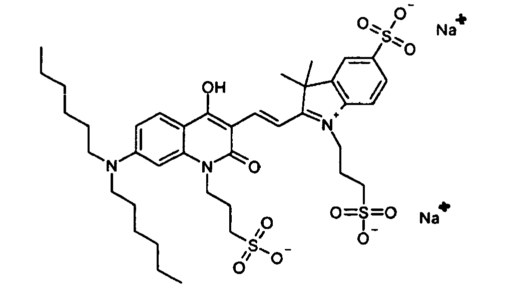 |
| 2 | 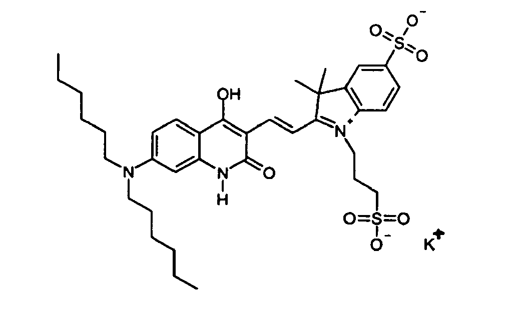 |
| 3 | 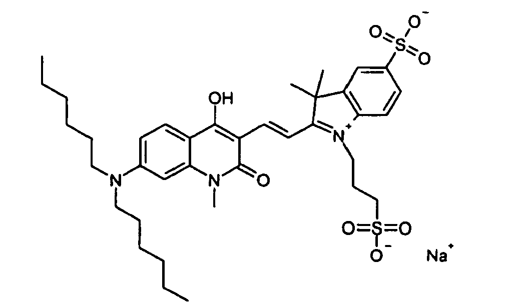 |

## Claims

1. A method for the fluorescence optical detection of proteins, **characterised in that** an aqueous mixture is used, which consists of the proteins to be detected, various detergents and one or more marker dyes on the basis of polymethines of the following general formulas **I-III** 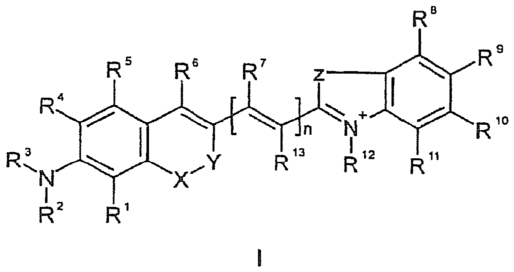 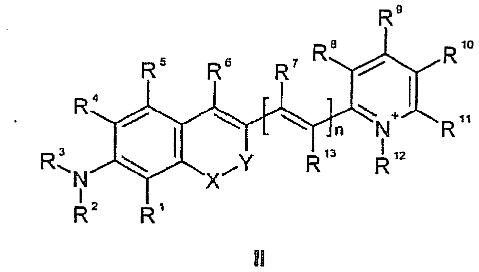 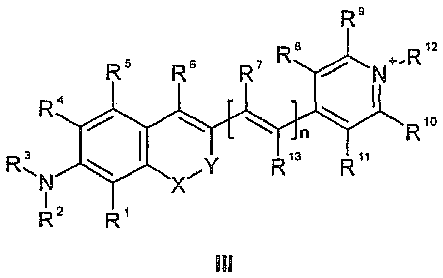
• wherein R¹ as well as R⁴ - R¹³ are identical or different and may be hydrogen, one or more chlorine and/or bromine atoms, alkyl, aryl, heteroaryl, cycloalkyl, heterocycloalkyl, alkyloxy, alkylmercapto, aryloxy, arylmercapto, heteroaryloxy, heteroarylmercapto, or cyano radicals, one or more hydroxyl, one or more alkyl-substituted or cyclic amino functions, and R² and R³ R³ are identical or different alkyl radicals comprising at least 3 carbon atoms, and R¹ and R² and/or R³ and R⁴ or R² and R³ and/or R⁸ and R⁹ and/or R¹⁰ and R¹¹ may form further aliphatic or aromatic rings or two adjacent radicals, e.g. R¹² and R¹³ in **I** or **II**, together may form one or more aliphatic or aromatic rings;
• X-Y together may represent an element selected from the group consisting of O, S, Se, Te, or the structural element (CR₂)ₘ, NR or SO₂, wherein R may identically or differently represent the functions of R¹ - R¹³ and m may be 1-4,
• X-Y may represent the structural elements -CR₂-O-, -O-CR₂-, -CO-O-, -CO-NR- or -NR-CO-, wherein R may identically or differently represent the functions of R¹-R¹³,
• Z in **I** may represent O, S, NR¹⁴ or CR¹⁵R¹⁶, wherein R¹⁴, R¹⁵ and R¹⁶ may identically or differently represent the functions of R¹ - R¹³, and
• n may represent a value from 0 - 4 and/or
• one or more of the substituents R¹ - R¹³ may represent a solubilising or ionisable or ionised substituent, such as SO₃⁻, PO₃²⁻, CO₂⁻, O⁻, NR₃⁺, cyclodextrin or sugar, and determines the hydrophilic properties of the dyes, and said substituents may also be attached to the basic chromophore itself via an aliphatic or heteroaliphatic, optionally cyclic, spacer group, and
• are **characterised in that** the aliphatic or heteroaliphatic spacer group consists of a structural element -[(CH₂)ₐ-Y-(CH₂)_{b}]_{c}-, wherein Y is identical with or different from a CR₂-, O-, S-, SO₂, SO₂NH-, NR-, CO₂- or CONR function, wherein R may represent the functions of R¹-R¹³ and wherein a and b, which are identical or different, represent values from 0-18 and c represents values from 1-18.

2. The method according to claim 1, wherein by joining the marker dyes of types I - III and the protein a marker-protein complex is formed which can be detected by means of fluorescence-optical methods.

3. The method according to claims 1 and 2, wherein at least one of the compounds 1-3 of the following formulas is/are used for the detection of proteins:
| No. | Formula |
|---|---|
| 1 | 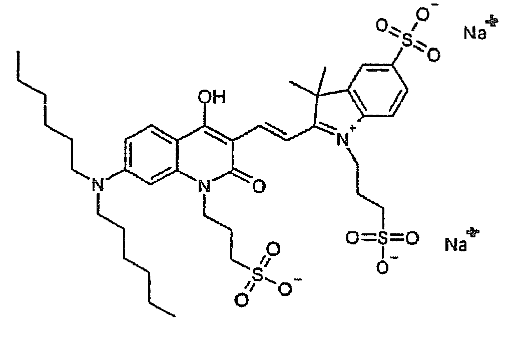 |
| 2 | 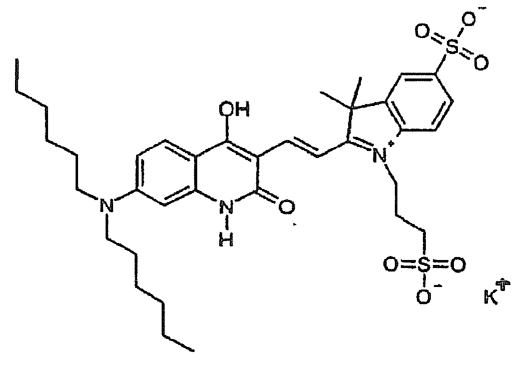 |
| 3 | 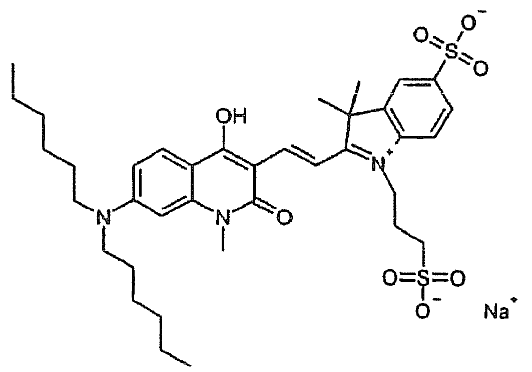 |

## Revendications

1. Procédé de détection de protéines par fluorescence optique, **caractérisé en ce que** l'on utilise une mixture aqueuse consistant des protéines à détecter, de divers détergents et d'un ou plusieurs colorants de marquage à base de polyméthines selon les formules générales suivantes **I-III** 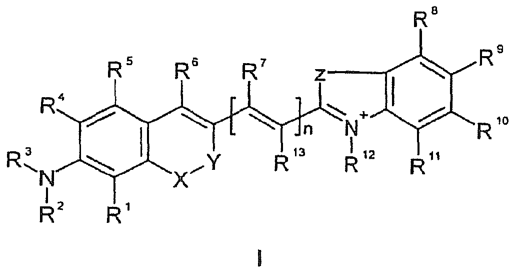 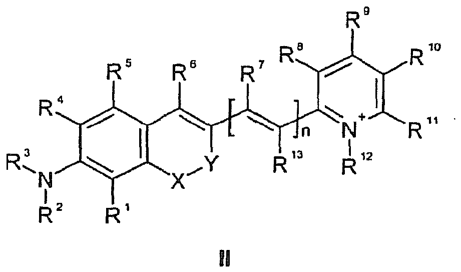 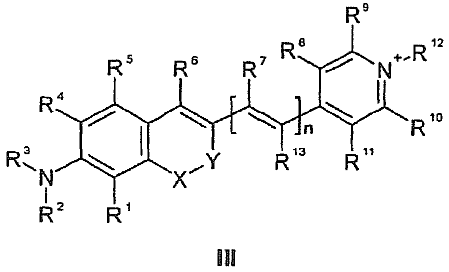
• dans lesquelles R¹ ainsi que R⁴ - R¹³, identiques ou différents, peuvent être l'hydrogène, un ou plusieurs atomes de chlore et/ou de brome, un radical alkyle, aryle, hétéroaryle, cycloalkyle, hétérocycloalkyle, alkyloxy, alkylmercapto, aryloxy, arylmercapto, hétéroaryloxy, hétéroarylmercapto ou cyano, une ou plusieurs fonctions hydroxy, une ou plusieurs fonctions amino substituées par un alkyle ou cycliques, et R² et R³, identiques ou différents, sont des radicaux alkyle présentant au moins 3 atomes de carbone, et R¹ et R² et/ou R³ et R⁴ ou R² et R³ et/ou R⁸ et R⁹ et/ou R¹⁰ et R¹¹ peuvent former d'autres cycles aliphatiques ou aromatiques, ou deux radicaux adjacents, par exemple R¹² et R¹³ dans **I** et **II**, peuvent former ensemble un ou plusieurs cycles aliphatiques ou aromatiques;
• X-Y peuvent représenter ensemble un élément choisi dans le groupe consistant en O, S, Se, Te, ou un élément structurel (CR₂)ₘ, NR ou SO₂, dans lequel R, identique ou différent, peut représenter les fonctions de R¹ - R¹³ et m peut être 1-4,
• X-Y peuvent représenter les éléments structurels -CR₂-O-, -O-CR₂-, -CO-O-, -CO-NR- ou -NR-CO-, dans lesquels R, identique ou différent, peut représenter les fonctions de R¹-R¹³,
• Z dans **I** peut représenter O, S, NR¹⁴ ou CR¹⁵R¹⁶, où R¹⁴, R¹⁵ et R¹⁶, identiques ou différents, peuvent représenter les fonctions de R¹ - R¹³, et
• n peut représenter une valeur de 0 - 4 et/ou
• un ou plusieurs des substituants R¹ - R¹³ peuvent représenter des substituants solubilisants ou ionisables ou ionisés, tel que SO₃⁻, PO₃²⁻, CO₂⁻, O⁻, NR₃⁺, cyclodextrine ou un sucre, qui déterminent les propriétés hydrophiliques des colorants, et lesdits substituants peuvent également être liés au chromophore de base lui-même par le biais d'un groupe espaceur aliphatique ou hétéroaliphatique ou optionnellement cyclique, et
• sont **caractérisés en ce que** le groupe espaceur aliphatique ou hétéroaliphatique consiste d'un élément structurel -[(CH₂)ₐ-Y-(CH₂)_{b}]_{c}-, dans lequel Y est identique avec ou différent d'une fonction CR₂-, O-, S-, SO₂, SO₂NH-, NR-, CO₂- ou CONR, où R peut représenter les fonctions de R¹-R¹³ , et dans lequel a et b, identiques ou différents, représentent les valeurs de 0-18, et c représente les valeurs de 1-18.

2. Procédé selon la revendication 1, dans lequel un complexe marqueur/protéine est formé par alliage des colorants de marquage du type I - III avec la protéine, ce complexe pouvant être détecté par des procédés de fluorescence optique.

3. Procédé selon les revendications 1 et 2, dans lequel on utilise pour la détection de protéines au moins un des composés 1-3 selon les formules suivantes:
| N° | Formule |
|---|---|
| 1 | 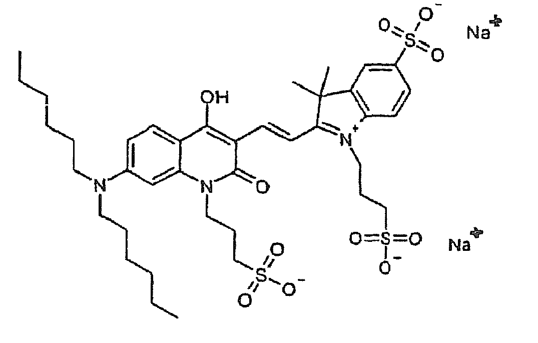 |
| 2 | 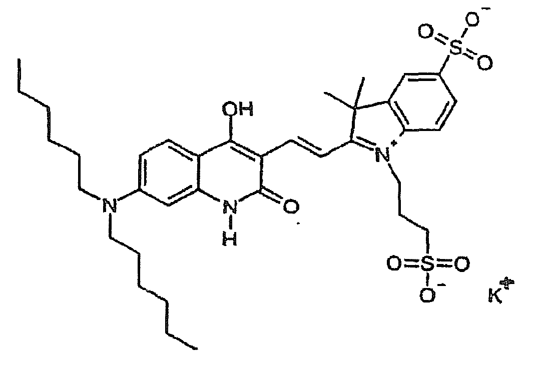 |
| 3 | 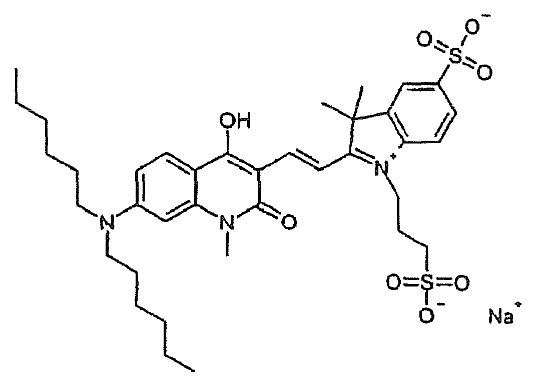 |
